# FASCICULE DE BREVET EUROPEEN

(11) **EP 1 025 901 B1**
(45) Date de publication et mention de la délivrance du brevet: **18.09.2002**
(21) Numéro de dépôt: 99403271.2
(22) Date de dépôt: 23.12.1999
(51) Int. Cl.: B01J 13/04, A61K 7/00, A61K 9/51

(54) **Nanocapsules à base de polymères anioniques hydrodispersibles, leur procédé de préparation et compositions cosmétiques ou dermatologiques les contenant**
Nanokapseln auf Basis von anionischen wasserdispergierbaren Polymeren, ihr Herstellungsverfahren und diese enthaltende kosmetische oder dermatologische Zusammensetzungen
Nanocapsules based on anionic hydrodispersible polymers , their fabrication process and cosmetic and dermatological compositons containing them

(30) Priorité: 29.12.1998 FR 9816553
(43) Date de publication de la demande: 09.08.2000
(73) Titulaire: L'OREAL, 75008 Paris (FR)
(72) Inventeur: Richart, Pascal, 75013 Paris (FR); Simonnet, Jean-Thierry, 75011 Paris (FR)
(74) Mandataire: Casalonga, Axel

(56) Documents cités:
- EP-A- 0 254 447
- EP-A- 0 274 961
- EP-A- 0 447 318
- EP-A- 0 676 451
- FR-A- 2 681 248
- GB-A- 1 243 454
- US-A- 3 947 571

## Description

La présente invention concerne des nanocapsules ayant une enveloppe constituée d'au moins un polymère anionique hydrodispersible, leur procédé de préparation ainsi que des compositions cosmétiques ou dermatologiques les contenant.

L'encapsulation ou l'absorption de principes actifs liphophiles dans des particules de dimensions submicroniques est connue depuis plusieurs années et est largement utilisée en particulier dans les domaines cosmétologique et dermatologique. En effet, ces particules appelées nanoparticules sont capables de traverser les couches superficielles du *stratum corneum* et de pénétrer dans les couches supérieures de l'épiderme vivant pour y libérer le principe actif. Cette pénétration dans des couches plus profondes élargit l'espace d'action des principes actifs et les met à l'abri d'une élimination rapide par simple frottement.

Le terme de "nanoparticules" englobe principalement deux systèmes différents : des "nanosphères" constituées d'une matrice polymérique poreuse dans laquelle le principe actif est absorbé et/ou adsorbé, ainsi que des "nanocapsules" ayant une structure de type noyau-enveloppe, c'est-à-dire une structure constituée d'un coeur lipidique formant ou contenant le principe actif, lequel coeur est encapsulé dans une enveloppe protectrice continue insoluble dans l'eau. La présente invention concerne uniquement ce deuxième type vésiculaire de nanoparticules, c'est-à-dire des nanocapsules à noyau lipidique entouré d'une membrane polymère.

La fabrication des nanocapsules a été décrite dans de nombreux documents. Elle se fait généralement soit par polymérisation interfaciale du ou des monomères formant le polymère de l'enveloppe (voir brevets BE-A-808 034, BE-A-839 748, BE-A-869 107, FR-A-2 504408 et FR-A-2 515 960), soit par émulsion de deux phases dont l'une contient le polymère formant l'enveloppe sous forme dissoute dans un solvant organique (voir par exemple EP-A-0 274 961, EP-A-557 489, EP-A-0-447 318 et WO 93/25195).

Le procédé utilisé de préférence par la demanderesse (EP-A-0 274 961, EP-A-0-447 318) consiste
- à dissoudre dans un solvant organique approprié un polymère, une phase lipidique formant ou contenant un principe actif et éventuellement un agent d'enrobage,
- à préparer une solution aqueuse d'un agent tensioactif approprié,
- à verser la phase organique dans la phase aqueuse tout en agitant modérément celle-ci,
- puis à évaporer la phase organique et, éventuellement, une partie de la phase aqueuse, pour obtenir une suspension concentrée de nanocapsules.

Dans la demande FR 2 681 248, ce procédé est utilisé pour la préparation de nanocapsules à coeur lipidique et à enveloppe polymérique non-biodégradable.

Cette manière de procéder suppose donc que l'on dispose d'un solvant organique approprié, c'est-à-dire
- non toxique,
- miscible en toutes proportions avec l'eau,
- plus volatil que l'eau et
- qui doit en outre être capable de dissoudre à la fois le polymère formant l'enveloppe, la phase lipidique (principe actif lipophile et/ou phase huileuse) et, éventuellement, un agent tensioactif jouant le rôle d'agent d'enrobage des nanocapsules.

Un solvant remplissant toutes ces conditions n'existe pas toujours pour les polymères susceptibles d'être utilisés pour la préparation des nanocapsules. L'éventail des solvants appropriés disponibles limite donc le choix des polymères utilisables.

L'objectif ayant abouti à la présente invention était par conséquent de s'affranchir de la nécessité de trouver un solvant ou une combinaison de solvants appropriés à chaque polymère.

Cet objectif a été atteint grâce à la découverte surprenante que des nanocapsules ayant une enveloppe à base de polymères hydrodispersibles anioniques pouvaient être préparées selon un procédé utilisant non pas une *solution* du polymère *dans une phase organique* mais *une dispersion* d'un polymère anionique hydrodispersible *dans une phase aqueuse*.

Par polymère hydrodispersible, on entend selon la présente invention un polymère qui, lorsqu'il est dispersé dans l'eau, se met sous forme de petites particules ayant une taille moyenne comprise entre 10 et 300 nm et de préférence entre 10 et 100 nm.

Ces nouvelles nanocapsules et le nouveau procédé pour les préparer permettent de conserver les procédures et installations habituelles de préparation des nanoparticules tout en dispensant l'homme de métier de trouver un solvant organique approprié pour chaque polymère formant l'enveloppe.

Par ailleurs, le procédé d'encapsulation connu implique généralement le chauffage de la phase organique et/ou de la phase aqueuse à des températures comprises entre 35 et 70 °C. Un autre avantage important de la présente invention réside dans le fait que l'encapsulation selon le nouveau procédé peut se faire à température ambiante ce qui est particulièrement intéressant pour limiter la décomposition, en cours d'encapsulation, de principes actifs sensibles à l'oxydation et à la chaleur, tels que le rétinol.

La présente invention a donc pour objet des nanocapsules constituées d'un coeur lipidique formant ou contenant un principe actif lipophile et d'une enveloppe polymérique continue insoluble dans l'eau comprenant au moins polymère anionique hydrodispersible synthétique choisi parmi les polyesters, poly(ester amide), polyuréthannes et copolymères vinyliques, portant tous des fonctions acide carboxylique et/ou sulfonique, et/ou un polymère anionique hydrodispersible naturel choisi parmi la résine shellac, la gomme de sandaraque et les dammars.

L'invention a également pour objet un nouveau procédé de préparation des nanocapsules ci-dessus consistant à mélanger, sous agitation, une dispersion aqueuse d'un polymère anionique hydrodispersible, contenant un agent tensioactif à caractère hydrophile, avec une solution de la phase lipidique formant ou contenant le principe actif lipophile dans un solvant organique et à évaporer le solvant organique et éventuellement une partie de l'eau.

Elle a en outre pour objet une composition cosmétique et/ou dermatologique contenant, dans un support physiologiquement acceptable, des nanocapsules à base de polymères hydrodispersibles anioniques tels que définis ci-dessus.

D'autres objets apparaîtront à la lecture de la description et des exemples qui suivront.

Les polymères anioniques hydrodispersibles utilisés dans la préparation des nanocapsules de la présente invention peuvent être des polymères d'origine naturelle ou synthétique et peuvent être biodégradables ou non biodégradables selon l'application envisagée.

En effet, lorsqu'on recherche une libération immédiate ou progressive du principe actif dans l'épiderme, on choisira de préférence un polymère biodégradable. Par contre, dans des applications où l'on souhaite obtenir une action prolongée dans le temps, par exemple pour la protection de la peau par des filtres solaires, dans le cas de la coloration directe de la peau ou pour le traitement de la peau par des agents déodorants, humectants ou piégeurs de radicaux libres, il est préférable de choisir un polymère non-biodégradable qui s'éliminera entre autres par desquamation.

Les polymères anioniques hydrodispersibles synthétiques utilisables selon la présente invention englobent des polyesters, poly(ester amides), polyuréthannes et copolymères vinyliques portant tous des fonctions acide carboxylique et/ou sulfonique.

Les polyesters anioniques sont obtenus par polycondensation de diacides carboxyliques aliphatiques, cycloaliphatiques et/ou aromatiques et de diols ou polyols aliphatiques, cycloaliphatiques et/ou aromatiques, un certain nombre de ces diacides et diols portant en outre une fonction acide carboxylique ou acide sulfonique libre ou sous forme de sel.

Comme diacides carboxyliques, on peut citer l'acide succinique, l'acide glutarique, l'acide adipique, l'acide pimélique, l'acide subérique, l'acide sébacique, l'acide téréphtalique, l'acide isophtalique ou l'anhydride de celui-ci.

Comme diols aliphatiques, on peut citer l'éthylèneglycol, le diéthylèneglycol, le triéthylèneglycol et le tétraéthylèneglycol, le di(hydroxyméthyl)cyclohexane, le diméthylolpropane ou le 4,4'-(1-méthylpropylidène)-bisphénol.

Les monomères polyols sont par exemple le glycérol, le pentaérytritol, le sorbitol.

Les comonomères permettant d'introduire des groupements anioniques sont par exemple l'acide diméthylolpropionique, l'acide trimellitique ou l'anhydride mellitique, ou un composé diol ou diacide carboxylique portant en plus un groupe SO₃M où M représente un atome d'hydrogène ou un ion d'un métal alcalin, tel que le 1,5-dihydroxypentane-3-sulfonate de sodium ou le 1,3-dicarboxybenzène-5-sulfonate de sodium.

Les poly(ester amide) utilisables dans le procédé de l'invention ont une structure similaire à celle des polyesters décrits ci-dessus mais contiennent en plus des motifs dérivés d'une diamine telle que l'hexaméthylènediamine, la méta- ou para-phénylènediamine, ou d'un aminoalcool tel que la monoéthanolamine.

Selon un mode de réalisation préféré de l'invention, le polymère anionique hydrodispersible est choisi parmi les polyesters aromatiques, cycloaliphatiques et/ou aliphatiques portant des fonctions acide sulfonique, c'est-à-dire des copolyesters comportant au moins des motifs dérivés d'acide isophtalique, d'acide sulfo-aryldicarboxylique et de diéthylèneglycol. Parmi ceux-ci, on peut citer tout particulièrement les polyesters comprenant des motifs dérivés d'acide isophtalique, d'acide sulfo-isophtalique, de diéthylèneglycol et de 1,4-di(hydroxyméthyl)cyclohexane, tels que ceux commercialisés sous les dénominations AQ®29, AQ®38, AQ®48 ULTRA, AQ®55S, AQ®1350, AQ®1045, AQ®1950 et AQ®14000 par la société EASTMAN CHEMICAL.

Ces polyesters peuvent aussi contenir des motifs dérivés d'éthylèneglycol, de triéthylèneglycol et/ou de tétraéthylèneglycol et d'acide téréphtalique comme ceux commercialisés sous les dénominations POLYCARE®PS 20, POLYCARE®PS 30 et POLYCARE®PS 32 par la société RHONE POULENC.

La proportion de motifs dérivés d'acide sulfoisophtalique est comprise entre 2 et 20 % en poids.

Les polyuréthannes utilisables en tant que polymères anioniques hydrodispersibles sont par exemple des copolymères polyuréthannespoly(acide acrylique) ou les copolymères polyuréthanne-polyester ou poly(ester uréthanne) anioniques.

Les copolymères vinyliques utilisables en tant que polymères hydrodispersibles anioniques englobent notamment des polymères filmogènes utilisés couramment pour la préparation de compositions cosmétiques parmi lesquels on peut citer :
(i) les copolymères acétate de vinyle/acide crotonique polyéthoxylés tels que celui commercialisé sous la dénomination ARISTO-FLEX® A par la société HOECHST ;
(ii) les copolymères acétate de vinyle/acide crotonique tels que celui commercialisé sous la dénomination LUVISET® CA66 par la société BASF;
(iii) les terpolymères acétate de vinyle/acide crotonique/néodécanoate de vinyle tels que celui commercialisé sous la dénomination RÉSINE 28-29-30®, par la société NATIONAL STARCH ;
(iv) les copolymères N-octylacrylamide/méthacrylate de méthyle/méthacrylate d'hydroxypropyle/acide acrylique/méthacrylate de *tert*-butylaminoéthyle tels que celui commercialisé sous la dénomination de AMPHOMER® par la société NATIONAL STARCH.
(v) les copolymères alternés méthylvinyléther/anhydride maléique monoestérifiés par le butanol, tels que celui commercialisé sous la dénomination GANTREZ® ES 425 par la société GAF ;
(vi) les terpolymères acide acrylique/acrylate d'éthyle/N-*tert*-butylacrylamide tels que celui commercialisé sous la dénomination ULTRAHOLD® 8 par la société BASF;
(vii) les polymères répondant à la formule générale suivante 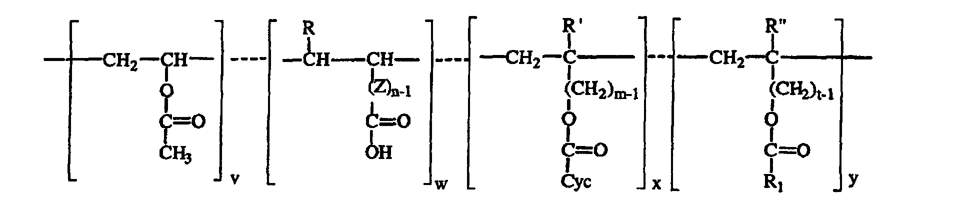 dans laquelle
   R, R' et R", identiques ou différents, représentent un atome d'hydrogène ou un radical méthyle,
   m, n et t valent 1 ou 2,
   R₁ représente un radical alkyle en C₂₋₂₁, linéaire ou ramifié, saturé ou insaturé,
   Z représente un radical divalent choisi parmi les résidus :
      -CH₂-, -CH₂-O-CH₂- et CH₂-O-(CH₂)₂-,
      *Cyc* représente un radical choisi parmi :
      (a) un radical de formule 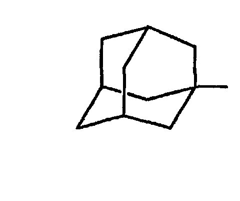
      (b) un radical de formule 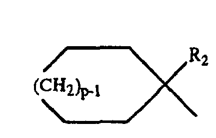 dans laquelle R₂ représente un atome d'hydrogène ou un radical méthyle, et p vaut 1 ou 2,
      (c) un radical de formule 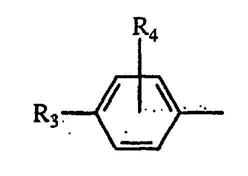 dans laquelle R₃ représente un atome d'hydrogène, un radical méthyle, éthyle, *tert*-butyle, éthoxy, butoxy ou dodécyloxy et R₄ représente un atome d'hydrogène, un radical alkyle en C₁₋₄ ou un radical alcoxy en C₁₋₄, et
      (d) un radical de formule 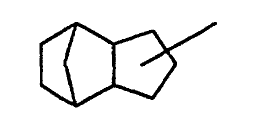
   v est choisi de manière à ce que les motifs correspondants représentent de 10 à 91 % en poids, de préférence de 36 à 84 % en poids du polymère total,
   w est choisi de manière à ce que les motifs correspondants représentent de 3 à 20 % en poids, de préférence de 6 à 12 % en poids du polymère total,
   x est choisi de manière à ce que les motifs correspondants représentent de 4 à 60 % en poids, de préférence de 6 à 40 % en poids du polymère total et
   y est choisi de manière à ce que les motifs correspondants représentent de 0 à 40 % en poids, de préférence de 4 à 30 % en poids du polymère total
   la somme de v + w + x + y étant égale à 100 %.

Parmi ces polymères on peut citer notamment le copolymère acétate de vinyle/*tert*-butyl-4-benzoate de vinyle/acide crotonique (65/25/10) neutralisé à 50 - 60 % par la lysine et le copolymère acétate de vinyle/acide crotonique/*tert*-butyl-4-benzoate de vinyle (65/10/25) neutralisé à 60 % par la lysine.

Comme polymères anioniques hydrodispersibles naturels susceptibles d'être utilisés selon la présente invention, on peut citer la résine shellac, la gomme de sandaraque et les dammars.

La résine shellac est une sécrétion animale, composée principalement de résine et de cire et est soluble dans certains solvants organiques. Elle doit être sous-neutralisée pour ne pas devenir soluble dans l'eau.

La gomme de sandaraque est une résine extraite de l'écorce d'arbres tels que le *thuya articulata* ou le *callitris verrucosa*. Elle est composée principalement d'acides tels que l'acide pimarique, l'acide callitrolique et l'acide sandaricinique. Elle est insoluble dans l'eau mais peut être solubilisée dans des solvants organiques tels que l'éthanol, l'acétone ou l'éther.

Les dammars sont des résines provenant d'arbres des genres Damara ou Shoréa et contiennent généralement 62,5 % de résènes (40 % de solubles et 22,5 % d'insolubles dans l'alcool) et 23 % d'acides.

La masse molaire moyenne en poids des polymères anioniques hydrodispersibles formant l'enveloppe des nanocapsules de la présente invention est généralement comprise entre 1000 et 5 000 000, de préférence entre 5000 et 500 000.

Les polymères hydrodispersibles anioniques décrits ci-dessus doivent être insolubles dans l'eau pour former efficacement une membrane continue insoluble. Or, la présence des charges anioniques, indispensable pour stabiliser la dispersion aqueuse des polymères, augmente leur polarité et favorise leur dissolution dans l'eau. Il est par conséquent indispensable de limiter le taux de charge des polymères.

Ce taux de charge limite supérieur qu'il convient de ne pas dépasser pour que le polymère reste insoluble dépend
- de la nature chimique du polymère, c'est-à-dire du caractère hydrophobe des motifs le composant,
- de la masse molaire du polymère, un polymère de faible masse molaire étant généralement plus soluble dans l'eau qu'un polymère de forte masse, ou encore
- de la nature de l'agent de neutralisation des fonctions acides.

Il est possible de modifier ce taux de charge en jouant sur la teneur en comonomères introduisant des fonctions acide carboxylique ou acide sulfonique ou sur le taux de neutralisation des groupements acide faible (groupements acide carboxylique).

La neutralisation partielle (sous-neutralisation) des fonctions d'acide faible, peut se faire par addition d'un agent monobasique non volatil, tel qu'une base minérale comme la soude ou la potasse, ou un aminoalcool pris dans le groupe constitué par l'amino-2-méthyl-2-propanol-1 (AMP), la triéthanolamine (TEA), la triisopropanolamine (TIPA), la monoéthanolamine, la diéthanolamine, la tri[(hydroxy-2)propyl-1]-amine, l'amino-2-méthyl-2-propanediol-1,3 (AMPD) et l'amino-2-hydroxyméthyl-2-propanediol-1,3.

On peut ainsi neutraliser environ 20 à 80 % des groupements ionisables pour stabiliser la dispersion aqueuse sans solubiliser le polymère.

L'utilisation des polymères anioniques hydrodispersibles précités pour l'encapsulation de principes actifs lipophiles a permis de mettre au point un nouveau procédé de préparation des nanocapsules. Ce nouveau procédé permet de s'affranchir de la nécessité de trouver un solvant approprié pour chaque polymère formant l'enveloppe des nanocapsules et peut en outre être réalisé à température ambiante ce qui le rend particulièrement approprié pour l'encapsulation de principes actifs liphophiles thermosensibles.

Le nouveau procédé de préparation de nanocapsules constituées d'un coeur lipidique formant ou contenant un principe actif et d'une enveloppe polymérique continue insoluble dans l'eau comprenant au moins un polymère anionique hydrodispersible, comprend les étapes suivantes consistant
(a) à dissoudre une phase lipidique formant ou contenant ledit principe actif lipophile et éventuellement un agent d'enrobage, dans un solvant organique approprié non toxique ayant un point d'ébullition inférieur à l'eau ;
(b) à préparer une dispersion aqueuse d'un des polymères anioniques hydrodispersibles précités ;
(c) à ajouter à la dispersion dudit polymère anionique dans l'eau obtenue dans l'étape (b) un agent tensioactif à caractère hydrophile non ionique, anionique ou cationique ;
(d) à réunir la phase organique de l'étape (a) et la phase aqueuse de l'étape (c) en maintenant une agitation modérée ; et
(e) à évaporer la totalité de la phase organique et, éventuellement, une partie de la phase aqueuse,
de manière à obtenir une suspension concentrée de nanocapsules.

Le solvant organique utilisé dans l'étape (a) pour dissoudre le principe actif lipophile et la phase lipidique le contenant peut être n'importe quel solvant organique non toxique plus volatil que l'eau utilisé couramment dans le domaine cosmétique. On utilise de préférence les cétones inférieures, notamment l'acétone, les alcools inférieurs, par exemple l'éthanol ou l'isopropanol, ou encore le méthylal et l'acétate d'éthyle, ou des mélanges de ces solvants.

Le polymère anionique hydrodispersible est dispersé dans la phase aqueuse de l'étape (b) à une concentration comprise entre 0,01 et 15 % en poids.

L'agent tensioactif dissous, dans l'étape (c) du procédé, dans la dispersion aqueuse du polymère est indispensable à l'obtention de nanocapsules sphériques ayant une distribution des tailles appropriée. Il assure en effet la stabilité des nanocapsules dans l'émulsion résultant du versement de la phase organique dans la phase aqueuse et prévient leur coalescence.

On peut utiliser n'importe quel agent tensioactif à caractère hydrophile, qu'il soit non-ionique, anionique ou cationique. On peut citer à titre d'exemple le laurylsulfate de sodium, les composés d'ammonium quaternaire, les monoesters de sorbitanne polyoxyéthylénés ou non, les éthers d'alcools gras et de polyoxyéthylèneglycol, les condensats d'oxyde d'éthylène et d'oxyde de propylène comme le produit PLURONIC® F-68 vendu par la société BASF ou les phospholipides tels que la lécithine.

On utilisera de préférence un agent tensioactif non ionique.

La concentration de l'agent tensioactif est déterminante pour le procédé de l'invention car elle détermine, parmi d'autres facteurs, la taille des nanocapsules obtenues. Le rapport pondéral de l'agent tensioactif aux matériaux constitutifs des nanocapsules est avantageusement compris entre 0,01 et 0,5 et de préférence voisin de 0,2.

Les nanocapsules selon l'invention constituées d'une enveloppe comprenant au moins un polymère anionique hydrodispersible spécifique présentent généralement une excellente étanchéité vis-à-vis du principe actif. Elles ne nécessitent par conséquent pas la présence d'un enrobage lamellaire qui est souvent indispensable pour empêcher la migration du principe actif lipophile contenu dans les nanocapsules de l'art antérieur vers une autre phase lipidique de la composition dans laquelle elles sont incorporées.

Dans certains cas, il peut cependant être nécessaire ou souhaitable de pourvoir les nanocapsules obtenues selon le procédé de la présente invention d'un tel enrobage lamellaire. Il s'agit d'une structure organisée en un ou plusieurs feuillet(s) lipidique(s) constitué(s) chacun d'une bicouche de molécules amphiphiles semblable à celle des membranes biologiques.

Les agents d'enrobage sont des agents tensioactifs à caractère hydrophobe, solubles dans la phase organique et qui sont capables, en présence d'eau, de former les doubles couches lipidiques décrites ci-dessus. Dans le procédé de la présente invention, cet agent d'enrobage est dissous dans la phase organique contenant le polymère et la phase lipidique.

On peut citer à titre d'exemple de tels agents d'enrobage les phospholipides tels que la lécithine selon la demande EP-A-447 318, certains polycondensats d'oxyde d'éthylène et d'oxyde de propylène comme les produits vendus sous la dénomination PLURONIC® par la société BASF tels que PLURONIC L121 ou sous la dénomination SYNPERONIC® par la société ICI, ou certains agents tensioactifs siliconés, tels que ceux décrits dans les documents US-A-5 364 633 et US-A-5 411 744 et utilisés dans la demande de brevet FR-A-2 742 677 (EP-A-780 115), par exemple ceux vendus par la société DOW CORNING sous les dénominations DC 5329, DC 7439-146, DC 2-5695 et Q4-3667.

Selon un mode de réalisation préféré de l'invention, les nanocapsules sont exemptes d'un tel enrobage lamellaire.

Les nanocapsules selon l'invention ont généralement une taille moyenne comprise entre 50 nm et 800 nm, de préférence entre 200 nm et 300 nm. La détermination de cette taille est réalisée par exemple à l'aide d'un granulomètre à laser (modèle Amtech BI 90 de la société Broohaven Instrument).

Elles peuvent contenir toutes sortes de principes actifs cosmétiques ou dermatologiques liphophiles.

On peut citer à titre d'exemple les agents émollients, les anti-inflammatoires, les anti-bactériens, les anti-fongiques, les anti-viraux, les anti-séborrhéiques, les anti-acnéiques, les kératolytiques, les anti-histaminiques, les anesthésiques, les agents cicatrisants, les modificateurs de la pigmentation, les filtres solaires, les piégeurs de radicaux libres, les agents hydratants, les vitamines et d'autres composés lipophiles similaires.

Selon la présente invention, le principe actif encapsulé est de préférence un principe actif lipophile sensible aux conditions physico-chimiques environnantes telles que la température, le pH, la lumière ou la présence d'agents oxydants.

On peut citer à titre d'exemples de principes actifs lipophiles préférés les vitamines telles que la vitamine A (rétinol) ou des esters de celle-ci, la vitamine E ou des esters de celle-ci tels que l'acétate de tocophérol, la vitamine D ou des dérivés de celle-ci et la vitamine F ou des dérivés de celle-ci, les carotènes tels que le β-carotène ou des dérivés de ceux-ci tels que le lycopène, et l'acide salicylique ou ses dérivés, notamment ceux décrits dans les documents FR-A-2 581 542, EP-A-378 936 et EP-A-570230, en particulier les acides n-octanoyl-5-salicylique, n-décanoyl-5-salicylique, n-dodécanoyl-5-salicylique, n-octyl-5-salicylique, n-heptyloxy-5-salicylique et n-heptyloxy-4-salicylique.

On a obtenu d'excellents résultats notamment pour l'encapsulation du rétinol (vitamine A), molécule très sensible à l'oxydation à pH acide, ainsi que pour les esters en C₁₋₃₀, plus particulièrement en C₁₋₆, de celui-ci, tels que l'acétate de rétinol et le propionate de rétinol.

Les nanocapsules de la présente invention ayant une enveloppe insoluble à base d'au moins un polymère anionique hydrodispersible peuvent, bien entendu, également être préparées selon le procédé de l'art antérieur décrit dans EP-A-0 274 961 et consistant
- à dissoudre le polymère anionique hydrodispersible, la phase lipidique formant ou contenant le principe actif lipophile et éventuellement un agent tensioactif jouant le rôle d'agent d'enrobage dans un solvant organique approprié, c'est-à-dire miscible avec l'eau, non toxique et plus volatil que l'eau,
- à préparer une solution d'un agent tensioactif approprié dans de l'eau,
- à verser la phase organique dans la phase aqueuse tout en agitant modérément celle-ci, ce qui aboutit à la formation spontanée d'une émulsion de nanocapsules,
- puis à évaporer la phase organique et, éventuellement, une partie de la phase aqueuse, pour obtenir une suspension concentrée de nanocapsules dans une phase aqueuse.

Les solvants organiques et agents tensioactifs hydrophiles et hydrophobes utilisés de préférence pour ce procédé d'encapsulation sont les mêmes que ceux décrits ci-dessus pour le nouveau procédé.

La fraction que représentent les nanocapsules dans les compositions cosmétiques et/ou dermatologiques de la présente invention est généralement comprise entre 0,1 et 30 % en poids et de préférence entre 0,5 et 15 % en poids, rapporté au poids total de la composition.

Les compositions peuvent contenir, en plus des nanocapsules et de la phase aqueuse, des adjuvants cosmétiques et/ou pharmaceutiques connus tels que des corps gras, de la vaseline, des agents régulateurs de pH, des conservateurs, des agents épaississants, des colorants ou des parfums.

Bien entendu, l'homme de métier veillera à choisir ce ou ces éventuels composés supplémentaires et leur quantité de manière à ce que les propriétés avantageuses attachées intrinsèquement à la composition cosmétique ou dermatologique conforme à l'invention ne soient pas, ou substantiellement pas, altérées par la ou les adjonctions envisagées.

Les compositions selon l'invention peuvent se présenter par exemple sous forme de sérum, de lotion, de gel aqueux, hydroalcoolique ou huileux, d'émulsion eau-dans-huile ou huile-dans-eau ou encore sous forme de dispersions aqueuses de vésicules lipidiques constituées de lipides ioniques ou non-ioniques ou d'un mélange de ceux-ci, lesquelles vésicules renferment ou non une phase huileuse.

Les exemples, donnés ci-après à titre purement illustratif , permettront de mieux comprendre l'invention.

### Exemple 1

Dans un ballon de 1 litre en verre ambré, sous atmosphère inerte, on disperse 1,0 g d'un copolyester constitué de motifs dérivés d'isophtalate/sulfo-isophtalate de sodium/1,4-dihydroxyméthylcyclohexane/diéthylèneglycol (AQ 55S commercialisé par la société Eastman Chemical) dans 300 g d'eau distillée à une température de 65 - 70 °C, puis on refroidit à température ambiante.

On ajoute à cette dispersion aqueuse 0,5 g de Pluronic® F68 vendu par la société BASF qui est un agent tensioactif non ionique à base d'oxyde d'éthylène et d'oxyde de propylène.

D'autre part, dans un ballon ambré de 500 ml, on dissout 5 g de triglycérides d'acide caprylique et d'acide caprique contenant 10 % de rétinol dans 200 ml d'acétone à température ambiante.

On verse ensuite la phase acétonique dans la phase aqueuse en maintenant une agitation vigoureuse.

On évapore ensuite l'acétone et une partie de l'eau avec un évaporateur rotatif jusqu'à l'obtention de 100 ml de solution.

On obtient ainsi une suspension colloïdale à pH 7,2 de nanocapsules ayant un diamètre moyen de 270 nm.

### Exemple 2

Dans un bêcher de 300 ml, on dissout 5 g de d'acétate de vitamine E dans 200 ml d'acétone.

Dans un second bêcher, on dissout 1 g d'un copolyester constitué de motifs dérivés de téréphtalate/isophtalate/sulfo-isophtalate de sodium/éthylèneglycol ayant une masse molaire moyenne en poids de 60 000 (POLYCARE PS 32 de la société RHÔNE POULENC) dans 300 g d'eau distillée à une température de 65 - 70 °C, puis on refroidit à température ambiante.

On ajoute à cette dispersion aqueuse 0,5 g de Pluronic® F68 vendu par la société BASF qui est un agent tensioactif non ionique à base d'oxyde d'éthylène et d'oxyde de propylène.

On verse ensuite la phase acétonique dans la phase aqueuse en maintenant une agitation vigoureuse.

On évapore ensuite l'acétone et une partie de l'eau avec un évaporateur rotatif jusqu'à l'obtention de 100 ml de solution.

On obtient ainsi une suspension colloïdale à pH 6,7 de nanocapsules ayant un diamètre moyen de 240 nm.

## Revendications

1. Nanocapsules avant une taille moyenne comprise entre 50 et 800 nm constituées
- d'un coeur lipidique formant ou contenant un principe actif lipophile et
- d'une enveloppe polymérique continue insoluble dans l'eau, **caractérisées par le fait que** ladite enveloppe polymérique comprend au moins un polymère anionique hydrodispersible synthétique qui, lorsqu'il est dispersé dans l'eau, se met sous forme de petites particules ayant une taille moyenne comprise entre 10 et 300 nm, choisi parmi les polyesters, poly(ester amides) et polyuréthannes, portant tous des fonctions acide carboxylique et/ou sulfonique, et/ou un polymère anionique hydrodispersible naturel qui, lorsqu'il est dispersé dans l'eau, se met sous forme de petites particules ayant une taille moyenne comprise entre 10 et 300 nm, choisi parmi la résine shellac, la gomme de sandaraque et les dammars.

2. Nanocapsules selon la revendication 1, **caractérisées par le fait que** ledit polymère anionique hydrodispersible synthétique est un polyester aromatique, çycloaliphatique et/ou aliphatique portant des fonctions acide sulfonique.

3. Nanocapsules selon la revendication 2, **caractérisées par le fait que** ledit polymère anionique hydrodispersible synthétique est un copolyester constitué de motifs dérivés d'acide isophtalique, d'acide sulfo-isophtalique, de diéthylèneglycol et de 1,4-di(hydroxyméthyl)-cyclohexane.

4. Nanocapsules selon la revendication 2, **caractérisées par le fait que** ledit polymère anionique hydrodispersible synthétique est un copolyester constitué de motifs dérivés d'acide isophtalique, d'acide sulfo-isophtalique, d'éthylèneglycol et d'acide téréphtalate.

5. Nanocapsules selon la revendication 3 ou 4, **caractérisées par le fait que** la proportion de motifs dérivés d'acide sulfo-isophtalique est comprise entre 2 et 20 % en poids.

6. Nanocapsules selon l'une quelconque des revendications précédentes, **caractérisées par le fait que** le polymère anionique hydrodispersible a une masse molaire moyenne en poids comprise entre 1000 et 5 000 000, de préférence entre 5000 et 500 000.

7. Nanocapsules selon l'une quelconque des revendications précédentes, **caractérisées par le fait qu'**elles ont une taille moyenne comprise entre 200 et 300 nm.

8. Nanocapsules selon l'une quelconque des revendications précédentes, **caractérisées par le fait que** le principe actif lipophile encapsulé est choisis parmi les agents émollients, les anti-inflammatoires, les anti-bactériens, les anti-fongiques, les anti-viraux, les anti-séborrhéiques, les anti-acnéiques, les kératolytiques, les anti-histaminiques, les anesthésiques, les agents cicatrisants, les modificateurs de la pigmentation, les filtres solaires, les piégeurs de radicaux libres, les agents hydratants et les vitamines.

9. Nanocapsules selon la revendication 8, **caractérisées par le fait que** le principe actif lipophile encapsulé est choisi parmi les principes actifs lipophiles sensibles aux conditions physico-chimiques environnantes telles que la température, le pH, la lumière ou la présence d'agents oxydants.

10. Nanocapsules selon la revendication 9, **caractérisées par le fait que** le principe actif lipophile est choisi parmi les vitamines telles que la vitamine A (rétinol), la vitamine E, la vitamine D et la vitamine F et les esters et dérivés de celles-ci, les carotènes tels que le β-carotène et les dérivés de ceux-ci tels que le lycopène, et l'acide salicylique et les dérivés de celui-ci, en particulier les acides n-octanoyl-5-salicylique, n-décanoyl-5-salicylique, n-dodécanoyl-5-salicylique, n-octyl-5-salicylique, n-heptyloxy-5-salicylique et n-heptyloxy-4-salicylique.

11. Nanocapsules selon la revendication 10, **caractérisées par le fait que** le principe actif lipophile est le rétinol ou un ester en C₁₋₃₀, plus particulièrement en C₁₋₆, de celui-ci, tel que l'acétate de rétinol ou le propionate de rétinol.

12. Composition cosmétique ou dermatologique, **caractérisée par le fait qu'**elle comprend, dans un support physiologiquement acceptable, des nanocapsules selon l'une quelconque des revendications précédentes.

13. Composition selon la revendication 12, **caractérisée par le fait qu'**elle comprend de 0,1 à 30 % en poids, de préférence de 0,5 à 15 % en poids, de nanocapsules, sur la base du poids total de la composition.

14. Procédé de préparation de nanocapsules constituées d'un coeur lipidique formant ou contenant un principe actif et d'une envelope polymérique continue insoluble dans l'eau comprenant au moins un polymère anionique hydrodispersible, **caractérisé par le fait qu'**il comprend les étapes consistant
(a) à dissoudre une phase lipidique formant ou contenant ledit principe actif lipophile, et éventuellement un agent d'enrobage, dans un solvant organique approprié non toxique ayant un point d'ébullition inférieur à l'eau ;
(b) à préparer une dispersion aqueuse d'un polymère anionique hydrodispersible synthétique, qui, lorsqu'il est dispersé dans l'eau, se met sous forme de petites particules ayant une taille moyenne comprise entre 10 et 300 nm, choisi parmi les polyesters, poly(ester amides), polyuréthannes et copolymères vinyliques, portant tous des fonctions acide carboxylique et/ou sulfonique, et/ou un polymère anionique hydrodispersible naturel choisi parmi la résine shellac, la gomme de sandaraque et les dammars;
(c) à ajouter un agent tensioactif à caractère hydrophile à la dispersion dudit polymère anionique dans l'eau obtenue dans l'étape (b) ;
(d) à réunir la phase organique de l'étape (a) et la phase aqueuse de l'étape (c) en maintenant une agitation modérée ; et
(e) à évaporer la totalité de la phase organique et, éventuellement, une partie de la phase aqueuse
pour obtenir une supension concentrée de nanocapsules.

15. Procédé selon la revendication 14, **caractérisé par le fait que** la concentration en polymère anionique hydrodispersible dans la dispersion de l'étape (b) est comprise entre 0,01 et 15 % en poids.

16. Procédé selon la revendication 14 ou 15, **caractérisé par le fait que** le solvant organique utilisé dans l'étape (a) est choisi parmi les cétones, notamment l'acétone, les alcools inférieurs tels que l'éthanol et l'isopropanol, l'acétate d'éthyle, le méthylal, et des mélanges de ceux-ci.

17. Procédé selon l'une quelconque des revendications 14 à 16, **caractérisé par le fait que** l'agent tensioactif est un agent tensioactif à caractère hydrophile non ionique, anionique ou cationique, de préférence un agent tensioactif non ionique.

18. Procédé selon la revendication 17, **caractérisé par le fait que** le rapport pondéral dudit agent tensioactif hydrophile aux matériaux constitutifs des nanocapsules est compris entre 0,01 et 0,5 et de préférence voisin de 0,2.

19. Procédé de préparation des nanocapsules selon l'une quelconque des revendications 1 à 11, consistant
- à dissoudre un polymère, une phase lipidique formant ou contenant un principe actif lipophile et éventuellement un agent d'enrobage dans un solvant organique miscible à l'eau approprié,
- à préparer une solution aqueuse d'un agent tensioactif approprié,
- à verser la phase organique dans la phase aqueuse tout en agitant modérément celle-ci,
- puis à évaporer la phase organique et, éventuellement, une partie de la phase aqueuse,
procédé de préparation **caractérisé par le fait que** le polymère utilisé dans la première étape est un polymère anionique hydrodispersible décrit dans l'une quelconque des revendications 1 à 6.

## Patentansprüche

1. Nanokapseln mit einer mittleren Größe im Bereich von 50 bis 800 nm, die aus
- einem Lipidkern, der einen lipophilen Wirkstoff bildet oder einen lipophilen Wirkstoff enthält, und
- einer in Wasser unlöslichen, kontinuierlichen Polymerhülle bestehen,
**dadurch gekennzeichnet, daß** die Polymerhülle mindestens ein synthetisches, in Wasser dispergierbares, anionisches Polymer, das in Form von kleinen Partikeln mit einer mittleren Größe von 10 bis 300 nm vorliegt, wenn es in Wasser dispergiert wird, und das unter den Polyestern, Poly(esteramiden) und Polyurethanen ausgewählt ist, die alle Carbonsäurefunktionen und/oder Sulfonsäurefunktionen tragen, und/oder ein natürliches, in Wasser dispergierbares, anionisches Polymer enthält, das in Form von kleinen Partikeln mit einer mittleren Größe von 10 bis 300 nm vorliegt, wenn es in Wasser dispergiert wird, und das unter Schellack, Sandarak und den Dammarharzen ausgewählt ist.

2. Nanokapseln nach Anspruch 1, **dadurch gekennzeichnet, daß** das synthetische, in Wasser dispergierbare, anionische Polymer ein aromatischer, cycloaliphatischer und/oder aliphatischer Polyester mit Sulfonsäurefunktionen ist.

3. Nanokapseln nach Anspruch 2, **dadurch gekennzeichnet, daß** das synthetische, in Wasser dispergierbare, anionische Polymer ein Copolyester ist, der aus Einheiten besteht, die von Isophthalsäure, Sulfoisophthalsäure, Diethylenglykol und 1,4-Di(hydroxymethyl)cyclohexan abgeleitet sind.

4. Nanokapseln nach Anspruch 2, **dadurch gekennzeichnet, daß** das synthetische, in Wasser dispergierbare, anionische Polymer ein Copolyester ist, der aus Einheiten besteht, die von Isophthalsäure, Sulfoisophthalsäure, Ethylenglykol und Terephthalsäure abgeleitet sind.

5. Nanokapseln nach Anspruch 3 oder 4, **dadurch gekennzeichnet, daß** der Mengenanteil der von Sulfoisophthalsäure abgeleiteten Einheiten im Bereich von 2 bis 20 Gew.-% liegt.

6. Nanokapseln nach einem der vorhergehenden Ansprüche, **dadurch gekennzeichnet, daß** das in Wasser dispergierbare, anionische Polymer ein Gewichtsmittel des Molekulargewichts im Bereich von 1000 bis 5 000 000 und vorzugsweise 5000 bis 500 000 aufweist.

7. Nanokapseln nach einem der vorhergehenden Ansprüche, **dadurch gekennzeichnet, daß** sie eine mittlere Größe im Bereich von 200 bis 300 nm aufweisen.

8. Nanokapseln nach einem der vorhergehenden Ansprüche, **dadurch gekennzeichnet, daß** der eingekapselte lipophile Wirkstoff unter den Emollientien, entzündungshemmenden Wirkstoffen, antibakteriellen Wirkstoffen, Fungiziden, antiviralen Wirkstoffen, Wirkstoffen gegen Seborrhoe, Wirkstoffen gegen Akne, Keratolytika, Antihistaminika, Anästhetika, Narbenbildnern, Wirkstoffen, die die Pigmentierung verändern, Sonnenschutzfiltern, Radikalfängern für freie Radikale, Hydratisierungsmitteln und Vitaminen ausgewählt ist.

9. Nanokapseln nach Anspruch 8, **dadurch gekennzeichnet, daß** der eingekapselte lipophile Wirkstoff unter den lipophilen Wirkstoffen ausgewählt ist, die gegenüber physikalisch-chemischen Bedingungen der Umgebung empfindlich sind, wie beispielsweise Temperatur, pH-Wert, Licht oder der Gegenwart von oxidierenden Stoffen.

10. Nanokapseln nach Anspruch 9, **dadurch gekennzeichnet, daß** der lipophile Wirkstoff unter den Vitaminen, wie Vitamin A (Retinol), Vitamin E, Vitamin D und Vitamin F und den Estern und Derivaten dieser Vitamine, den Carotinen, wie β-Carotin und ihren Derivaten, wie Lycopin, und Salicylsäure und ihren Derivaten, insbesondere 5-n-Octanoylsalicylsäure, 5-n-Decanoylsäuresylicylsäure, 5-n-Dodecanoylsalicylsäure, 5-n-Octylsalicylsäure, 5-n-Heptyloxysalicylsäure und 4-n-Heptyloxysalicylsäure ausgewählt ist.

11. Nanokapseln nach Anspruch 10, **dadurch gekennzeichnet, daß** der lipophile Wirkstoff das Retinol oder ein C₁₋₃₀-Ester und besonders ein C₁₋₆-Ester von Retinol ist, wie Retinolacetat oder Retinolpropionat.

12. Kosmetische oder dermatologische Zusammensetzung, **dadurch gekennzeichnet, daß** sie in einem physiologisch akzeptablen Träger die Nanokapseln nach einem der vorhergehenden Ansprüche enthält.

13. Zusammensetzung nach Anspruch 12, **dadurch gekennzeichnet, daß** sie 0,1 bis 30 Gew.-% und vorzugsweise 0,5 bis 15 Gew.-% Nanokapseln, bezogen auf das Gesamtgewicht der Zusammensetzung, enthält.

14. Verfahren zur Herstellung von Nanokapseln, die aus einem Lipidkern, der einen Wirkstoff bildet oder einen Wirkstoff enthält, und einer in Wasser unlöslichen, kontinuierlichen Polymerhülle, die mindestens ein in Wasser dispergierbares anionisches Polymer enthält, bestehen, **dadurch gekennzeichnet, daß** es die folgenden Schritte umfaßt:
(a) eine Lipidphase, die den lipophilen Wirkstoff und gegebenenfalls ein Mittel zum Umhüllen enthält oder diese bildet, wird in einem geeigneten, nicht toxischen, organischen Lösungsmittel gelöst, das einen Siedepunkt unter dem Siedepunkt von Wasser aufweist;
(b) es wird eine wäßrige Dispersion eines synthetischen, in Wasser dispergierbaren, anionischen Polymers, das in Form von kleinen Partikeln mit einer mittleren Größe im Bereich von 10 bis 300 nm vorliegt, wenn es in Wasser dispergiert wird, und das unter den Polyestern, Poly(esteramiden), Polyurethanen und Vinylcopolymeren ausgewählt ist, die alle Carbonsäurefunktionen und/oder Sulfonsäurefunktionen aufweisen, und/oder eines natürlichen, in Wasser dispergierbaren, anionischen Polymers, das unter Schellack, Sandarak und Dammarharzen ausgewählt ist, hergestellt;
(c) in die Dispersion des anionischen Polymers in Wasser, die in Schritt (b) hergestellt wurde, wird ein grenzflächenaktiver Stoff mit hydrophilem Charakter eingearbeitet;
(d) die organische Phase des Schritts (a) und die wäßrige Phase des Schritts (c) werden vereinigt, wobei mäßig gerührt wird; und
(e) die gesamte organische Phase und gegebenenfalls ein Teil der wäßrigen Phase wird verdampft,
um eine konzentrierte Suspension von Nanokapseln herzustellen.

15. Verfahren nach Anspruch 14, **dadurch gekennzeichnet, daß** die Konzentration des in Wasser dispergierbaren anionischen Polymers der Dispersion des Schritts (b) im Bereich von 0,01 bis 15 Gew.-% liegt.

16. Verfahren nach Anspruch 14 oder 15, **dadurch gekennzeichnet, daß** das in Schritt (a) verwendete organische Lösungsmittel unter den Ketonen, insbesondere Aceton, den niederen Alkoholen, wie Ethanol und Isopropanol, Ethylacetat, Methylal und deren Gemischen ausgewählt ist.

17. Verfahren nach einem der Ansprüche 14 bis 16, **dadurch gekennzeichnet, daß** der grenzflächenaktive Stoff ein nichtionischer, anionischer oder kationischer grenzflächenaktiver Stoff mit hydrophilem Charakter und vorzugsweise ein nichtionischer grenzflächenaktiver Stoff mit hydrophilem Charakter ist.

18. Verfahren nach Anspruch 17, **dadurch gekennzeichnet, daß** das Gewichtsverhältnis des hydrophilen grenzflächenaktiven Stoffes und der Stoffe, die die Nanokapseln aufbauen, im Bereich von 0,01 bis 0,5 und vorzugsweise in der Gegend von 0,2 liegt.

19. Verfahren zur Herstellung von Nanokapseln nach einem der Ansprüche 1 bis 11, das darin besteht
- ein Polymer, eine Lipidphase, die einen lipophilen Wirkstoff und gegebenenfalls ein Mittel zum Umhüllen enthält oder diese bildet, in einem geeigneten, mit Wasser mischbaren organischen Lösungsmittel zu lösen,
- eine wäßrige Lösung eines geeigneten grenzflächenaktiven Stoffes herzustellen,
- die organische Phase unter mäßigem Rühren in die wäßrige Phase zu gießen,
- und anschließend die organische Phase und gegebenenfalls einen Teil der wäßrigen Phase zu verdampfen,
**dadurch gekennzeichnet, daß** das Polymer, das in dem ersten Schritt eingesetzt wird, ein in Wasser dispergierbares, anionisches Polymer nach einem der Ansprüche 1 bis 6 ist.

## Claims

1. Nanocapsules with an average size of between 50 and 800 nm consisting
- of a lipid centre forming or containing a lipophilic active principle, and
- of a water-insoluble continuous polymeric envelope, **characterized in that** the said polymeric envelope comprises at least one synthetic water-dispersible anionic polymer which, when dispersed in water, takes the form of small particles with an average size of between 10 and 300 nm, chosen from polyesters, poly(ester amides) and polyurethanes, all bearing carboxylic and/or sulphonic acid functions and/or a natural water-dispersible anionic polymer which, when dispersed in water, takes the form of small particles with an average size of between 10 and 300 nm, chosen from shellac resin, sandarac gum and dammar resins.

2. Nanocapsules according to Claim 1, **characterized in that** the said synthetic water-dispersible anionic polymer is an aromatic, cycloaliphatic and/or aliphatic polyester bearing sulphonic acid functions.

3. Nanocapsules according to Claim 2, **characterized in that** the said synthetic water-dispersible anionic polymer is a copolyester consisting of units derived from isophthalic acid, from sulphoisophthalic acid, from diethylene glycol and from 1,4-di(hydroxymethyl)cyclohexane.

4. Nanocapsules according to Claim 2, **characterized in that** the said synthetic water-dispersible anionic polymer is a copolyester consisting of units derived from isophthalic acid, from sulphoisophthalic acid, from ethylene glycol and from terephthalic acid.

5. Nanocapsules according to Claim 3 or 4,
**characterized in that** the proportion of units derived from sulphoisophthalic acid is between 2 and 20% by weight.

6. Nanocapsules according to any one of the preceding claims, **characterized in that** the water-dispersible anionic polymer has a weight-average molar mass of between 1000 and 5,000,000, preferably between 5000 and 500,000.

7. Nanocapsules according to any one of the preceding claims, **characterized in that** they have an average size of between 200 and 300 nm.

8. Nanocapsules according to any one of the preceding claims, **characterized in that** the encapsulated lipophilic active principle is chosen from emollients, anti-inflammatory agents, antibacterial agents, antifungal agents, antiviral agents, anti-seborrhoeic agents, anti-acne agents, keratolytic agents, anti-histaminic agents, anaesthetics, cicatrizing agents, pigmentation modifiers, sunscreens, free-radical scavengers, moisturizers and vitamins.

9. Nanocapsules according to Claim 8, **characterized in that** the encapsulated lipophilic active principle is chosen from lipophilic active principles which are sensitive to the surrounding physicochemical conditions such as the temperature, pH, light or the presence of oxidizing agents.

10. Nanocapsules according to Claim 9, **characterized in that** the lipophilic active principle is chosen from vitamins such as vitamin A (retinol), vitamin E, vitamin D and vitamin F, or esters or derivatives thereof, carotenes such as β-carotene or derivatives thereof, for instance lycopene, and salicylic acid or derivatives thereof, in particular 5-n-octanoylsalicylic acid, 5-n-decanoylsalicylic acid, 5-n-dodecanoylsalicylic acid, 5-n-octylsalicylic acid, 5-n-heptyloxysalicylic acid and 4-n-heptyloxysalicylic acid.

11. Nanocapsules according to Claim 10, **characterized in that** the lipophilic active principle is retinol or a C₁-C₃₀, more particularly C₁-C₆, ester thereof, such as retinyl acetate or retinyl propionate.

12. Cosmetic or dermatological composition, **characterized in that** it comprises, in a physiologically acceptable support, nanocapsules according to any one of the preceding claims.

13. Composition according to Claim 12, **characterized in that** it comprises from 0.1 to 30% by weight, preferably from 0.5 to 15% by weight, of nanocapsules, based on the total weight of the composition.

14. Process for preparing nanocapsules consisting of a lipid centre forming or containing an active principle, and a water-insoluble continuous polymeric envelope comprising at least one water-dispersible anionic polymer, **characterized in that** it comprises the steps consisting
(a) in dissolving a lipid phase forming or containing the said lipophilic active principle and optionally a coating agent, in a suitable nontoxic organic solvent which has a boiling point below that of water;
(b) in preparing an aqueous dispersion of a synthetic water-dispersible anionic polymers which, when dispersed in water, takes the form of small particles with an average size of between 10 and 300 nm, chosen from polyesters, poly(ester amides), polyurethanes and vinyl copolymers, all bearing carboxylic and/or sulphonic acid functions and/or a natural water-dispersible anionic polymer chosen from shellac resin, sandarac gum and dammar resins, [sic];
(c) in adding a surfactant of hydrophilic nature to the dispersion of the said anionic polymer in the water obtained in step (b);
(d) in combining the organic phase from step (a) and the aqueous phase from step (c) while maintaining moderate stirring; and
(e) in evaporating all of the organic phase and, possibly, some of the aqueous phase,
so as to obtain a concentrated suspension of nanocapsules.

15. Process according to Claim 14, **characterized in that** the concentration of water-dispersible anionic polymer in the dispersion of step (b) is between 0.01 and 15% by weight.

16. Process according to Claim 14 or 15, **characterized in that** the organic solvent used in step (a) is chosen from ketones, in particular acetone, lower alcohols such as ethanol and isopropanol, ethyl acetate and methylal, and mixtures thereof.

17. Process according to any one of Claims 14 to 16, **characterized in that** the surfactant is a surfactant of nonionic, anionic or cationic hydrophilic nature, preferably a nonionic surfactant.

18. Process according to Claim 17, **characterized in that** the weight ratio of the said hydrophilic surfactant to the materials constituting the nanocapsules is between 0.01 and 0.5 and preferably in the region of 0.2.

19. Process for preparing the nanocapsules according to any one of Claims 1 to 11, which consists
- in dissolving a polymer, a lipid phase forming or containing a lipophilic active principle, and optionally a coating agent in a suitable water-miscible organic solvent,
- in preparing an aqueous solution of a suitable surfactant,
- in adding the organic phase to the aqueous phase with moderate stirring,
- and then in evaporating the organic phase and, possibly, some of the aqueous phase,
this preparation process being **characterized in that** the polymer used in the first step is a water-dispersible anionic polymer described in any one of Claims 1 to 6.
